Europäisches Patentamt

European Patent Office

Office européen des brevets

(11) Publication number: **0 357 292**
**A1**

## (12) EUROPEAN PATENT APPLICATION

(21) Application number: **89308316.2**

(22) Date of filing: **16.08.89**

(51) Int. Cl.⁵: **C07D 301/10 , B01J 23/50**

(30) Priority: **30.08.88 GB 8820500**
**07.09.88 GB 8820975**
**12.10.88 GB 8823919**
**16.12.88 GB 8829343**

(43) Date of publication of application:
**07.03.90 Bulletin 90/10**

(84) Designated Contracting States:
**BE DE FR GB IT NL SE**

(71) Applicant: **IMPERIAL CHEMICAL INDUSTRIES PLC**
**Imperial Chemical House Millbank**
**London SW1P 3JF(GB)**

(72) Inventor: **Hayden, Percy**
**2 Hawthorne Drive Hutton Meadows**
**Guisborough Cleveland(GB)**
Inventor: **Jennings, James Robert**
**9 Rudby Lea Hutton Rudby**
**Yarm Cleveland(GB)**

(74) Representative: **Locke, Timothy John et al**
**Imperial Chemical Industries PLC Legal**
**Department: Patents Bessemer Road PO Box**
**6**
**Welwyn Garden City Hertfordshire, AL7**
**1HD(GB)**

(54) **Production of ethylene oxide and catalysts therefor.**

(57) In a process in which ethylene oxide is produced by contacting a gas stream comprising ethylene and oxygen with a silver containing catalyst which is also contacted with a chlorine containing reaction modifier and an oxide of nitrogen, more ethylene oxide is produced at similar selectivity if the gas phase comprises more than 35% ethylene by volume.

EP 0 357 292 A1

# EP 0 357 292 A1

**THIS INVENTION relates to the production of ethylene oxide.**

In our European patent No 3642 we have disclosed a process of producing an olefine oxide which comprises contacting an olefine with oxygen in the presence of an elemental silver-containing catalyst and a chlorine containing reaction modifier in which the performance of the catalyst is improved by contacting the catalyst with a nitrate or nitrite forming substance which is in a gas phase simultaneously with the chlorine containing reaction modifier.

We have now found that in a process of this type, when both a chlorine containing reaction modifier and a nitrate or nitrite forming substance is present more ethylene oxide is produced under otherwise comparable conditions at a similar selectivity (ie with a similar number of moles of ethylene oxide produced per hundred moles of ethylene consumed) if the gas phase comprises more than 35% ethylene by volume.

This invention comprises a process in which ethylene oxide is produced by contacting a gas stream comprising ethylene and oxygen, with a silver containing catalyst which is also contacted with a chlorine containing reaction modifier and an oxide of nitrogen selected from $NO_2$, $N_2O_4$, NO and $N_2O_3$ by means of the gas stream characterised in that the gas stream comprises 35 to 92% preferably more than 40% to 85% and more preferably 43 to 60% by volume of ethylene.

The gas stream preferably comprises 0.5 to 200 parts per million by volume of the oxide of nitrogen, which may be provided as a substance which is converted to the said oxide of nitrogen under the process conditions.

Organic compounds capable on oxidation of producing the oxide of nitrogen may be used as such substances for example esters of nitrous acid for example methyl nitrite, nitroso compounds for example N-nitroso compounds, nitroso butane, organic nitro compounds especially the nitro paraffins having 1-4 carbon atoms for example nitropropane, nitroaromatic compounds, especially nitrobenzene, di-nitro compounds, nitriles, for example acetonitrile, HCN, amides for example formamide or amines for example $C_1$ to $C_4$ alkylamines especially methylamine or ethylene diamine. Ammonia, hydrazine and/or hydroxylamine may also be used as such a substance.

The catalyst suitably comprises silver supported on a porous heat resisting support which has a specific surface area in the range 0.05 to 10 $m^2/g$ and preferably 0.1 to 5$m^2/g$ and more preferably 0.3 to 5 $m^2/g$ as measured by the Brunauer, Emmett and Teller method.

The support is suitably a preformed support.

The silver may be introduced to a preformed porous heat resisting support as a suspension of silver or silver oxide in a liquid medium for example water or by impregnation of the support with a solution of a silver compound which can be reduced to silver metal if necessary by means of a reducing agent for example hydrogen. If necessary a heat treatment may be used to decompose the silver compound to silver. Suitably the impregnating solution contains a reducing agent which may be for example an anion, for example a formate, acetate, propionate, lactate, oxalate or tartarate ion, of a silver compound in the solution. The reducing agent may be for example an aldehyde, for example formaldehyde or acetaldehyde or an alcohol preferably having 1 to 4 carbon atoms for example methanol or ethanol.

The solution may be a solution in water and/or an organic solvent, for example an aliphatic alcohol preferably having 1 to 4 carbon atoms; a polyhydric alcohol for example ethylene glycol or glycerol; a ketone for example acetone; an ether for example dioxan or tetrahydrofuran; a carboxylic acid for example acetic acid or preferably molten lactic acid which is preferably used in the presence of water and suitably in the presence of an oxidising agent for example $H_2O_2$; or an ester for example ethyl acetate or a nitrogen containing base for example pyridine or formamide. An organic solvent may function as a reducing agent and/or complexing agent for the silver also.

If the silver is introduced by impregnating a support with a solution of a decomposable silver compound it is preferred that ammonia and/or a nitrogen containing base should be present. The nitrogen containing base suitably acts as a ligand maintaining the silver in solution; for example it may be pyridine, acetonitrile, an amine, especially a primary or secondary amine having 1-6 carbon atoms, or preferably ammonia. Other suitable nitrogen-containing bases include acrylonitrile, hydroxylamine and alkanolamines for example ethanolamine, alkylene diamines having from 2-4 carbon atoms or polyamines containing at least three carbon atoms for example diethylenetriamine or amino ethers with at least one ether linkage and at least one primary or secondary amino group eg morpholine or amides for example formamide or dimethyl formamide. The nitrogen-containing bases may be used alone or in admixture. They also act as reducing agents for the silver compound. Suitably the nitrogen containing base or bases are used together with water.

Alternatively the solution may be a neutral or acid solution, for example it may be a solution of a silver

2

carboxylate especially a formate, acetate, propionate, oxalate, citrate, tartarate or preferably lactate or for example a solution of silver nitrate.

The solutions preferably contain 3-50% of silver by weight.

Impregnation may be carried out in a single stage or if desired may be repeated one or more times. By this means higher silver contents of the catalyst may be achieved.

The silver compound may be decomposed for example in an atmosphere of air or nitrogen.

The silver compound may be partially or completely reduced to silver by heating in the range 100 to $350^\circ$ C, for example for a period of 5 mins to 4 hours; alternatively reduction may be carried out during the oxidation of the olefine to the olefine oxide.

The catalyst support preferably has an apparent porosity as measured by the mercury absorption method of at least 20%, for example 30-80% preferably 30-65% and more preferably 40-60% and mean pore diameters of 0.1 to 20 microns preferably 0.3 to 4 microns as measured by' the mercury porosimetry method. The pore size distribution of the support may be bimodal, in which case the smaller pores preferably account for at least 70% of the total pore volume and have a mean pore diameter preferably in the range of 0.1 and preferably 0.3 to 4 microns, and the larger pores preferably have a mean pore diameter in the range 25 to 500 microns.

Most of the silver content of the catalyst is preferably present in the form of discrete particles adhering to the support having equivalent diameters of less than 10,000 A preferably in the range 20-10,000A and more preferably 40-8,000A for example 100-5,000A. By equivalent diameter is meant the diameter of a sphere of the same silver content as the particle.

Preferably at least 80% of the silver is present as particles having equivalent diameters in the aforesaid range, the quantity of silver being judged in terms of the number of particles falling in that range. The silver is believed to be present largely as metallic silver. The dimensions of the silver particles may be determined by scanning electron microscopy.

The support may be an alumina, silicon carbide, silica, zirconia, titania or silica/alumina support, but it is preferably composed of an aggregate of alpha-alumina particles which may be fused together or cemented together with, for example silica or baryta.

The catalyst preferably comprises 3 to 50% and more preferably 3 to 30% for example 6 to 28% by weight of silver.

It is preferred that the catalyst should contain cations, for example alkali and/or alkaline earth metal cations as the corresponding nitrate or nitrite or in a form capable of reacting to produce the corresponding nitrate or nitrite. This is especially preferred if the catalyst is treated with the nitrate or nitrite forming substance intermittently in order to restore its selectivity. It may be that an effect of such cations is to hold more nitrate and/or nitrite ions on the catalyst or to hold them longer than is the case in their absence but we do not wish to be bound by any theory as to their action.

The cations may be introduced to the support before during or after the introduction of the silver compound. Suitably the cations are introduced to a support in which the silver is present in metallic form. The cations are suitably introduced as solutions in water and/or organic solvents. If it is desired to impregnate a catalyst which has already been used in the oxidation of an alkene to an alkylene oxide and has lost performance, this may be carried out also. Suitable concentrations of such cations in the catalyst in a form which is extractable by water may be for example $10^{-6}$ to $2 \times 10^{-4}$ and preferably $4 \times 10^{-6}$ to $10^{-4}$ gram equivalents per gram of catalyst.

The cations are preferably provided as nitrates, hydroxides, carbonates, bicarbonates or carboxylates.

The catalyst preferably contains as such cations potassium ions, as compound(s) which are extractable from the catalyst by water.

The total pressure in the process may be in the range of from 1 to 100 and preferably 3-100 bars absolute. The oxygen concentration should not exceed the flammable limit of about 8% by volume. The oxygen may be supplied for example in the form of air or preferably as commercial oxygen. A diluent for example helium, nitrogen, argon, and/or a lower paraffin for example ethane and/or preferably methane may be present in amounts of for example 5 to 52% and preferably 40 to 50% by volume in total. Suitably the diluent comprises methane as aforesaid together with, for example 100 to 100,000 parts per million by volume of ethane, preferably together with small amounts, for example 10 to 10,000 parts per million by volume of $C_3$ to $C_6$ alkanes, cycloalkanes or alkenes preferably propylene, cyclopropane, isobutene or isobutane.

It is necessary to operate using gas compositions which are outside the explosive limits.

The temperature is suitably in the range 180 to $320^\circ$ C, preferably 200 to $300^\circ$ C and more preferably in the range 220 to $290^\circ$ C. Contact times should be sufficient to convert 0.5 - 70%, for example 2 to 20 and preferably 5 - 20% of the ethylene and unconverted ethylene is, after separation of the product, suitably recycled, optionally in the presence of unconverted oxygen where appropriate and suitably after removal of $CO_2$.

Suitable catalysts for use in this invention may be as described in Netherlands Patent Applications 7705847 and 7805163.

The chlorine-containing reaction modifier may be of known type. It is preferred that it should be a $C_1$ to $C_{10}$ compound also containing hydrogen. It may be for example 1,1 or preferably, 1,2-dichlorethane methyl chloride or vinyl chloride. Chlorinated aromatic compounds, for example chlorobenzene, dichlorobenzene and chlorinated toluenes are also suitable. It is preferred that the concentration of the chlorine containing reaction modifier should be in the range 0.1 - 500 and preferably 1 to 50 parts per million parts of the reaction medium by weight.

The oxide of nitrogen may be supplied continuously to the process at a low level for example 0.1 and preferably 0.2 to 200 and preferably 0.5 to 50 parts per million of $NO_2$ equivalent of the process gas by volume.

As chlorine and nitrate and/or nitrile ions tend to be retained in the catalyst it is not essential to feed the chlorine containing reaction modifier and oxide of nitrogen continuously or simultaneously but it is preferred to feed both continuously so as to maintain the catalyst continuously in an optimum condition.

Examples

Preparation of Catalysts

a) Preparation of Reagents

1 Silver isopropylamine complex.
960 ml mono-isopropylamine were added over 3 hours to a mixture of 856 g $AgNO_3$ and 144 ml water. The temperature was maintained in the range 40-50°C during the addition and stirred for a further hour. The final volume of the reagent was brought to 1170 ml by addition of a further 100 ml isopropylamine to clarify the solution and to replace amine lost by volatilisation. The final silver content was 0.47 g/ml.

2 Lithium solution.
432 g analytical grade $LiNO_3$ $3H_2O$ was dissolved in 150 ml water to give a final volume of 460 ml.

3 Potassium solution.
22g analytical grade $K_2CO_3$ was warmed and shaken with a mixture of 41.7g 2-ethylhexanoic acid and 30 ml water. When the effervescence had subsided 1.7 litres of white spirit was added and the mixture refluxed to removed water using the Dean and Stark method. 48 ml 2-ethylhexanol was then added, and the mixture warmed to dissolved the solids. The mixture was filtered to remove any cloudiness and made up to two litres with white spirit. The potassium content of the resulting solution was 5 parts by weight of potassium per litre.

b) Preparation of Support

A solution of 4.39 g $Rb_2CO_3$ and 2g 40% v/v HF in water was made up to 932g with demineralised water, and sprinkled onto well-stirred boehmite (800g) sold under the trade name "Versal" 250. The stirring was continued for 10 minutes and the mixture was thoroughly mixed by repeated extrusion. The product was sealed for 3 hours prior to extrusion through one 8 mm die with a 3 mm core-rod. The product was dried for 16 hours at 120°C prior to firing. The firing cycle was as follows: Heating from ambient temperature at 200°C per hour to 700°C and holding for one hour at 700°C followed by an increase in termperature over 7 to 8 hours to 1600°C and holding for 1 hour at 1600°C prior to cooling.

The properties of the support were as follows:-

| Hollow cylinders | 8 mm x 6 mm with a 2 mm hole |
|---|---|
| Pore Volume | 1.12 ml/g |
| Surface Area (BET method) | 1.06 m²/g |
| Si Content | 580 ppm w/v |
| Sol. Na Content | 54 ppm w/v |
| Sol. K Content | 10 ppm w/v |

## c) Preparation of Precursor

70.6g of the support was vacuum impregnated with a mixed solution from 160 ml of the silver complex solution and 91.3 ml of the lithium nitrate solution. The catalyst particles were drained and pyrolysed under nitrogen at $300°C$ over a period of 5 hours to decompose the silver complex essentially to the metallic state. The product was washed in boiling water for 16 hours, calcined under nitrogen to $300°C$ and washed a second time. The silver content was 28%, and this precursor was used for the preparation of catalysts A, B and C.

## d) Preparation of Catalysts

The catalysts were prepared by impregnation of the precursor with solutions of potassium compound. From a knowledge of the pore volume, the concentration of potassium in the pores assuming them to be filled with solution as previously made up gives the "nominal potassium content after drying. Using the potassium stock solution described above, appropriate solutions were made by dilution with a mixture of 2-ethylhexanol and white spirit in a ratio of about 48:1700. Samples of precursor were then impregnated with the solutions for 16 hours in rocking vials, followed by draining and drying in air at $400°C$ for one hour. The catalyst preparation was then completed by calcination in air at $700°C$ for 14 hours. The catalysts prepared are shown in table 1.

Table 1

| | CATALYST A | CATALYST B | CATALYST C |
|---|---|---|---|
| Nominal K content | 120 ppm | 160 ppm | 200 ppm |

## Testing the Catalysts

The catalysts were crushed and sieved to produce particulate matter in the size range 425 to 1000 microns. An aliquot .75 was loaded into a middle section of a stainless steel reactor (length, 25.4 cms; internal diameter 4mm).

Gas mixtures of ethylene 29% and 43%, oxygen 8%, carbon dioxide 0.5% vinyl chloride (4.0 ppm), ethyl chloride (1.2 ppm) nitromethane 20 ppm with nitrogen to balance were passed at a pressure of 16 atmospheres absolute over the catalyst. The gas was passed at an hourly space velocity of 8400. All percentages and parts per million (ppm) of the gas mixture are by volume. The temperature of the reactor was adjusted to achieve 30% conversion of the oxygen fed : this is designated $T_{30}$.

The selectivity of the catalyst, S, is the number of moles of ethylene oxide produced expressed as a percentage of the number of moles of ethylene consumed. $S_{30}$ is the selectivity at 30% conversion of the oxygen fed. The results of testing catalyst A-C in gas mixtures containing 29% or 43% ethylene are given in Tables 2-4. It can be seen that the presence of the higher concentration of ethylene has resulted in

a) a lower value for the $T_{30}$, ie higher activity

b) a fall or lower rate of increase in the $T_{30}$ value, ie higher catalyst stability, and

c) at higher levels of potassium, no significant penalty in selectivity.

Table 2

| Catalyst A | | | | |
|---|---|---|---|---|
| Days on Line | 43% ethylene 120K | | 29% ethylene 120K | |
| | T30 | S30 | T30 | S30 |
| 1 | 231.6 | 85.9 | 240.2 | 85.2 |
| 2 | 225.9 | 84.8 | 236.5 | 85.2 |
| 3 | 226.7 | 84.4 | 235.4 | 85.2 |
| 4 | 226.2 | 84.2 | 234.2 | 85.2 |
| 5 | 227.4 | 84.2 | 234.1 | 85.2 |
| 6 | 226.9 | 84.2 | 235.3 | 85.3 |
| 7 | 226.5 | 84.1 | 234.5 | 85.4 |
| 8 | 225.9 | 84.3 | 234.1 | 85.4 |
| 9 | 227.0 | 84.3 | 235.1 | 85.4 |
| 10 | 227.8 | 84.1 | 235.3 | 85.4 |
| 11 | 227.3 | 84.3 | 235.5 | 85.5 |
| 12 | 227.8 | 84.3 | 235.9 | 85.5 |
| 13 | 228.0 | 84.5 | 236.3 | 85.7 |
| 14 | 228.3 | 84.3 | 236.9 | 85.6 |
| 15 | 229.1 | 84.2 | 237.1 | 85.6 |
| 16 | 230.1 | 84.3 | 238.0 | 85.5 |

Table 3

| Catalyst B | | | | |
|---|---|---|---|---|
| Days on Line | 29% ethylene 160K | | 43% ethylene 160K | |
| | T30 | S30 | T30 | S30 |
| 1 | 239.8 | 85.7 | 231.4 | 86.3 |
| 2 | 235.9 | 85.8 | 226.9 | 85.7 |
| 3 | 234.7 | 85.7 | 225.9 | 85.5 |
| 4 | 234.7 | 85.9 | 225.2 | 85.4 |
| 5 | 235.1 | 85.8 | 225.9 | 85.5 |
| 6 | 234.9 | 86.2 | 225.8 | 85.9 |
| 7 | 235.2 | 86.2 | 226.0 | 86.0 |
| 8 | 236.7 | 86.1 | 225.8 | 85.9 |
| 9 | 237.8 | 86.0 | 226.2 | 85.9 |
| 10 | 238.7 | 85.9 | 226.2 | 85.8 |
| 11 | 239.4 | 85.7 | 226.7 | 85.8 |
| 12 | 241.1 | 85.6 | 227.2 | 85.8 |
| 13 | 242.1 | 85.4 | 226.8 | 85.7 |
| 14 | 243.0 | 85.5 | 227.1 | 85.6 |
| 15 | 243.0 | 85.3 | 227.1 | 85.6 |
| 16 | 245.0 | 85.3 | 227.3 | 85.9 |
| 17 | 245.4 | 85.0 | 277.1 | 85.8 |
| 18 | 246.2 | 84.9 | 227.2 | 85.7 |

EP 0 357 292 A1

Table 4

| Catalyst C | | | | |
|---|---|---|---|---|
| Days on Line | 29% ethylene 200K | | 43% ethylene 200K | |
| | T30 | S30 | T30 | S30 |
| 1 | 232.5 | 84.6 | 229.0 | 86.6 |
| 2 | 234.0 | 86.7 | 226.9 | 86.6 |
| 3 | 234.1 | 86.8 | 226.3 | 86.4 |
| 4 | 236.8 | 86.5 | 226.2 | 86.1 |
| 5 | 238.7 | 86.5 | 226.1 | 85.9 |
| 6 | 239.1 | 86.4 | 226.4 | 85.7 |
| 7 | 240.1 | 86.1 | 226.2 | 85.7 |
| 8 | 241.9 | 85.7 | 226.5 | 85.7 |
| 9 | | | 227.4 | 85.7 |
| 10 | | | 225.2 | 83.8 |
| 11 | | | 226.9 | 85.3 |
| 12 | | | 227.4 | 85.2 |
| 13 | | | 227.7 | 85.4 |
| 14 | | | 227.9 | 85.3 |
| 15 | | | 228.1 | 85.0 |
| 16 | | | 227.9 | 84.9 |
| 17 | | | 228.3 | 85 |
| 18 | | | 228.1 | 84.7 |
| 19 | | | 227.6 | 84.7 |
| 20 | | | 238.6 | 84.6 |
| 21 | | | 235.1 | 85.2 |
| 22 | | | 231.8 | 85.5 |
| 23 | | | 228.9 | 85.1 |
| 24 | | | 230.8 | 84.9 |
| 25 | | | 230.6 | 85.1 |
| 26 | | | 232.1 | 85.3 |
| 27 | | | 231.6 | 85.1 |
| 28 | | | 231.9 | 85.1 |
| 29 | | | 232.3 | 85.1 |
| 30 | | | 234.5 | 85.6 |
| 31 | | | 233.4 | 85.8 |
| 32 | | | 234.7 | 86.0 |
| 33 | | | 236 | 86.1 |
| 34 | | | 236.1 | 86.1 |
| 35 | | | 236.4 | 86.1 |
| 36 | | | 236.8 | 86 |
| 37 | | | 237.1 | 85.8 |
| 38 | | | 237.6 | 85.6 |
| 39 | | | 237.2 | 85.9 |

## Claims

1. A process in which ethylene oxide is produced by contacting a gas stream comprising ethylene and oxygen with a silver containing catalyst which is also contacted with a chlorine containing reaction modifier and an oxide of nitrogen selected from $NO_2$, $N_2O_4$, $NO$ and $N_2O_3$ by means of the gas stream characterised in that the gas stream comprises 35 to 92% by volume of ethylene.

2. A process as claimed in claim 1 in which the chlorine containing reaction modifier and the oxide of

7

EP 0 357 292 A1

nitrogen are present substantially continuously in the gas stream.

3. A process as claimed in claim 1 or 2 in which the oxide of nitrogen is provided as a substance which is converted thereto under the process conditions.

4. A process as claimed in claim 1, 2 or 3 in which the gas stream comprises more than 40% of ethylene.

5. A process as claimed in any preceding claim in which the catalyst comprises 3 to 50% of silver deposited on a porous, heat resisting support.

6. A process as claimed in claim 5 in which the catalyst comprises $10^{-6}$ to $2 \times 10^{-4}$ gram equivalents of potassium cations in a form which is extractable by water per gram of catalyst.

7. A process as claimed in claim 6 in which the catalyst comprises at least $4 \times 10^{-6}$ gram equivalents of potassium cations in a form which is extractable by water per gram of catalyst.

8. A process as claimed in any preceding claim in which the chlorine containing reaction modifier is present in an amount of 0.1 to 500 parts per million parts of the gas stream and the oxide of nitrogen is present in an amount of 0.5 to 200 parts per million by volume of the gas stream.

9. A process as claimed in any preceding claim in which the total pressure is 3 to 100 bars absolute the oxygen concentration does not exceed about 8% by volume and the temperature is 200 to 300°C.

8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 161 480 (MITSUI TOATSU)<br>* Abstract; page 2, line 45 - page 4, line 5; claims 1-12 *<br>--- | 1-9 | C 07 D 301/10<br>B 01 J 23/50 |
| A | FR-A-2 534 581 (VASILEVICH et al.)<br>* Abstract; page 12, example 2 *<br>--- | | |
| A | US-A-4 376 209 (WATANABE et al.)<br>--- | | |
| D,A | EP-A-0 003 642 (I.C.I.)<br>----- | | |

TECHNICAL FIELDS SEARCHED (Int. Cl.5)

B 01 J
C 07 D

The present search report has been drawn up for all claims

| Place of search | Date of completion of the search | Examiner |
|---|---|---|
| THE HAGUE | 26-10-1989 | LO CONTE C. |